(19) Europäisches Patentamt
European Patent Office
Office européen des brevets

(11) **EP 4 073 744 B1**

(12) **EUROPEAN PATENT SPECIFICATION**

(45) Date of publication and mention
of the grant of the patent:
**25.03.2026 Bulletin 2026/13**

(21) Application number: **20829626.9**

(22) Date of filing: **11.12.2020**

(51) International Patent Classification (IPC):
**G06T 5/50** *(2006.01)* **G06T 7/00** *(2017.01)*
**G01V 5/00** *(2024.01)* **G01N 23/02** *(2006.01)*

(52) Cooperative Patent Classification (CPC):
**G06T 5/50; G01N 23/10; G01V 5/228; G01V 5/232;**
**G06T 7/0004;** G01N 2223/33; G01N 2223/401;
G01N 2223/639; G01N 2223/643;
G06T 2207/10116; G06T 2207/30112;
G06T 2207/30232

(86) International application number:
**PCT/GB2020/053189**

(87) International publication number:
**WO 2021/116704 (17.06.2021 Gazette 2021/24)**

(54) **CORRECTION OF IMAGES AND DEPTH INFORMATION FOR DETECTION WITH MATRIX**

KORREKTUR VON BILDERN UND TIEFENINFORMATIONEN ZUR DETEKTION MIT EINER MATRIX

CORRECTION D'IMAGES ET D'INFORMATIONS DE PROFONDEUR POUR DÉTECTION PAR MATRICE

(84) Designated Contracting States:
**AL AT BE BG CH CY CZ DE DK EE ES FI FR GR
HR HU IE IS IT LI LT LU LV MC MK MT NL NO PL
PT RO RS SE SI SK SM TR**

(30) Priority: **13.12.2019 GB 201918415**

(43) Date of publication of application:
**19.10.2022 Bulletin 2022/42**

(73) Proprietor: **Smiths Detection France S.A.S.
94400 Vitry-sur-Seine (FR)**

(72) Inventors:
• **MAITREJEAN, Serge
94400 Vitry-Sur-Seine (FR)**
• **GRABEUIL, Sébastien
94400 Vitry-Sur-Seine (FR)**
• **BERGERAT, Cindy
94400 Vitry-Sur-Seine (FR)**

(74) Representative: **Vienne, Aymeric Charles Emile et al
Mathys & Squire
The Shard
32 London Bridge Street
London SE1 9SG (GB)**

(56) References cited:
**US-A1- 2004 251 415     US-B1- 6 178 223**

## Description

### Field of Invention

The disclosure relates but is not limited to a method for inspecting cargo with X-rays. The disclosure also relates but is not limited to a corresponding inspection system and a corresponding computer product or a computer program.

### Background

Inspecting cargo with X-rays may be performed by scanning the cargo by mutually displacing the cargo and a scanner including a detector, with a mutual scanning displacement, and detecting, with the scanner, radiation generated by a plurality of successive X-ray pulses irradiating the cargo during the mutual scanning displacement. A matrix of detectors comprising a plurality of at least two rows of detectors may be used for inspection.

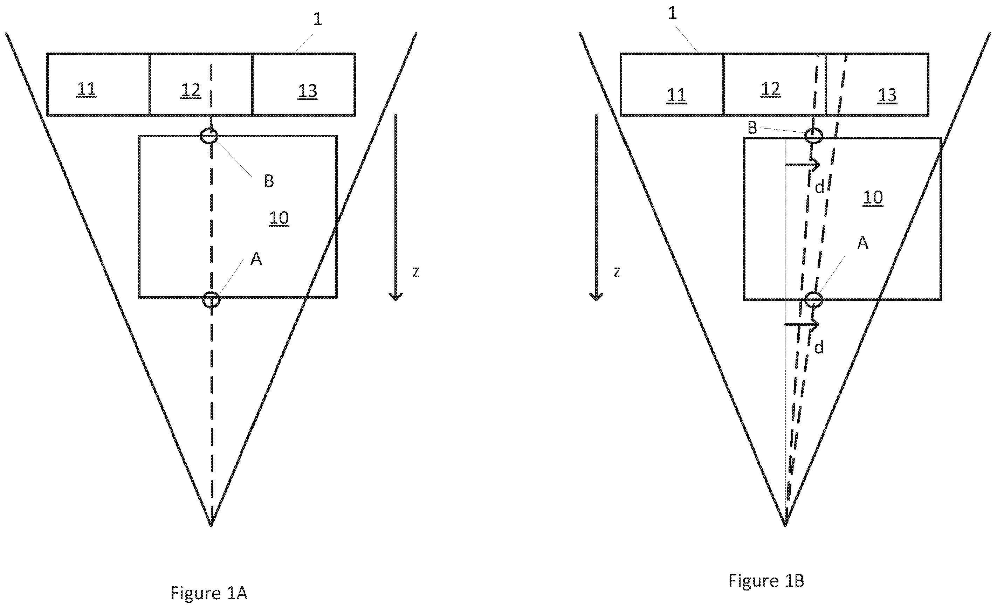

As illustrated in Figure 1A and Figure 1B, a projection of a point A or B of a cargo 10 on a matrix 1 depends on a distance z of the point A or B from the matrix 1. As illustrated in Figure 1A, points A and B are projected on a row 12 of the matrix 1. As illustrated in Figure 1B, when the point A located at a distance z corresponding to a middle plane is moving a distance d during the scan, the point A is projected on a row 13 of the matrix 1. However when the point B located at a distance z closer to the matrix 1 is moving the same distance d during the scan, the point B is projected on the row 12 of the matrix 1. It should be understood that the two points A and B which were situated on the same line of projection for one pulse on Figure 1A (and therefore represented by a single point in a corresponding image) are dissociated for the X-ray pulse of Figure 1B.. The dependency of the projections on depth introduces distortions in a corresponding final image of the cargo.

The distortions in the final image are also due to the fact that, for generating the final image of the cargo, the arrangement of data is performed with respect to a reconstruction plane. Selecting the reconstruction plane corresponds to considering that the whole cargo is located in the reconstruction plane. Therefore, zones of the cargo which are located in the reconstruction plane appear without distortion on the final image.

However zones of the cargo which are located in other planes than the reconstruction plane will appear with distortions in the final image because of the dissociation of points of the cargo explained above. Oversampling of data increases the above distortions, because some parts of the cargo are irradiated several times and will appear several times in the final image.

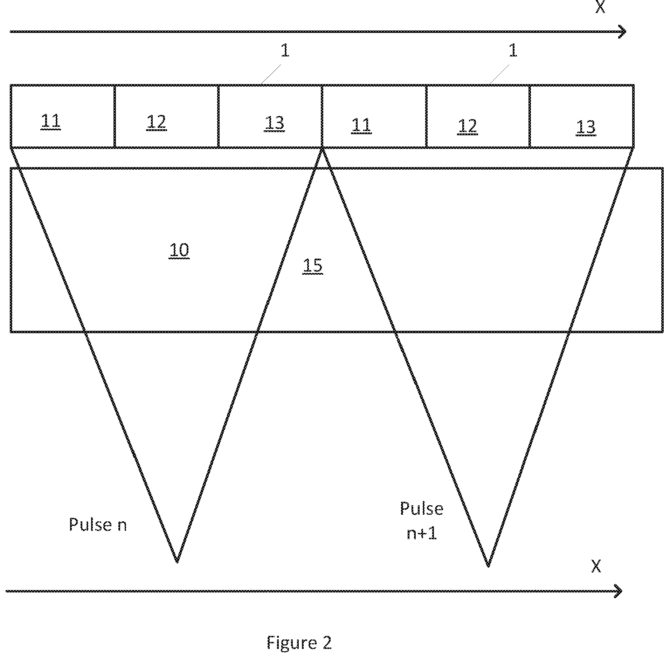

Figure 2 shows the matrix 1 moving and the cargo 10 not moving during the mutual scanning displacement. In other words Figure 2 corresponds to a mobile mode, but the same explanations would also apply to a pass-through mode. In order to avoid the oversampling mentioned above, as illustrated in Figure 2, the matrix 1 may be used such that a frequency of the X-ray pulses is adapted to a speed of the matrix 1 during the mutual scanning displacement x. The frequency of the X-ray pulses may be calculated such that a displacement of the matrix 1 between two pulses corresponds approximately to a width of the matrix 1 of detectors. However, adjusting the frequency of the X-ray pulses to the speed of the matrix 1 as just explained has as a consequence that zones 15 of the cargo 10 are not imaged.

Prior art document US 2004/251415 A1 discusses alternative methods of x-ray based cargo inspection for multiple planes at different distances from the source.

Summary of Invention

The invention is set out in the appended claims.

### Brief Description of Drawings

Embodiments of the present disclosure will now be described, by way of example, with reference to the accompanying drawings, in which:

Figure 1A, already discussed, schematically illustrates projections of points of a cargo irradiated by a first X-ray pulse, the projections being on a matrix of detectors;
Figure 1B, already discussed, schematically illustrates projections of the points of Figure 1A irradiated by a second X-ray pulse;
Figure 2, already discussed, schematically illustrates a cargo irradiated by a first X-ray pulse and a second X-ray pulse, the frequency of the pulses being selected to avoid oversampling;
Figure 3 shows a flow chart illustrating an example method according to the disclosure;
Figure 4 schematically illustrates a cargo irradiated by a first X-ray pulse and a second X-ray pulse, the frequency of the pulses being selected to avoid parts of the cargo not being imaged;
Figures 5A and 5B illustrate examples of artefacts depending on a choice for a plane of reconstruction for intermediate images;
Figure 6 illustrates an example neighbourhood according to the disclosure;
Figure 7 shows another flow chart illustrating an example method according to the disclosure;
Figure 8 schematically illustrates an example final image with a superimposed depth image; and
Figure 9 schematically illustrates an example system configured to implement a method according to any aspect of the present disclosure.

In the figures, similar elements bear identical numerical references.

## Specific Description of Example Embodiments

Overview

Embodiments of the disclosure provide a method for processing inspection data associated with cargo irradiated by a plurality M of successive pulses of X-rays. The inspection data is generated as a result of scanning the cargo using a matrix of detectors. The matrix comprises a plurality N of at least two rows of detectors. The successive pulses are generated by a source. The matrix is located at a distance D from the source. The scanning of the cargo comprises mutually displacing the cargo and the matrix with a mutual scanning displacement, and detecting, with the matrix, radiation corresponding to the plurality M of successive X-ray pulses irradiating the cargo, during the mutual scanning displacement.

In the inspection data, radiation corresponding to the plurality M of successive pulses irradiating the cargo is arranged in a first order on the plurality N of rows of detectors of the matrix. Successive reconstruction zones for the inspection data are then determined. Each reconstruction zone corresponds to a range of distances from the source in which radiation corresponding to the plurality M of successive pulses irradiating the cargo changes arrangement order. For each determined reconstruction zone, a corresponding intermediate image is generated, and an average image of the intermediate images is also generated. Zones of distortion are then identified in the average image, and, for each zone of distortion neighbourhoods are extracted from each intermediate image, and the neighbourhood having less distortion than the other extracted neighbourhoods is selected.

The selected neighbourhood may be used to correct the average image to generate a final image of the cargo, with reduced distortion.

The selected neighbourhood may be used to determine information relating to the corresponding reconstruction zone. The information may comprise an ordinal number corresponding to a relative position of the reconstruction zone in the one or more successive reconstruction zones and/or at least one distance from the source corresponding to at least one boundary of the range of distances associated with the reconstruction zone. The information may be used to generate a depth image providing information about a distance of parts of the cargo relative to the matrix.

Embodiments of the disclosure may enable correcting the average image to generate a final image of the cargo, with reduced distortion. Embodiments of the disclosure may enable generating a depth image providing information about a distance of parts of the cargo relative to the matrix and/or the source. Embodiments of the disclosure may enable covering the whole cargo and correct distortion due to the oversampling of some parts of the cargo.

Details Description of Example Embodiments

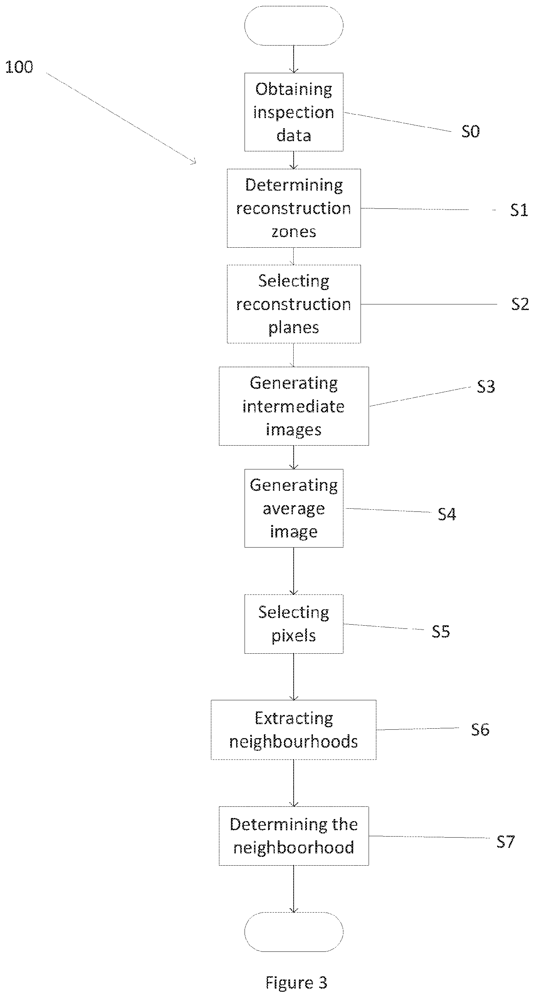

Figure 3 shows a flow chart illustrating an example method 100 according to the disclosure.

The method 100 is mainly for processing inspection data associated with cargo irradiated by a plurality M of successive pulses of X-rays. The method 100 illustrated in Figure 3 mainly comprises the following steps:

obtaining, at S0, the inspection data;
determining, at S1, one or more successive reconstruction zones for the inspection data;
selecting, at S2, one or more reconstruction planes, based on the determined one or more reconstruction zones;
for each selected reconstruction plane, generating, at S3, an intermediate image of the cargo;
generating, at S4, an average image by averaging all of the generated intermediate images;
on the generated average image, selecting, at S5, one or more pixels having a gradient greater than a predetermined threshold; and
for each one of the pixels selected at S5:

extracting, at S6, a neighbourhood of the selected pixel from each generated intermediate image, and
determining, at S7, in the extracted neighbourhoods, the neighbourhood minimizing a criterion compared with the other extracted neighbourhoods.

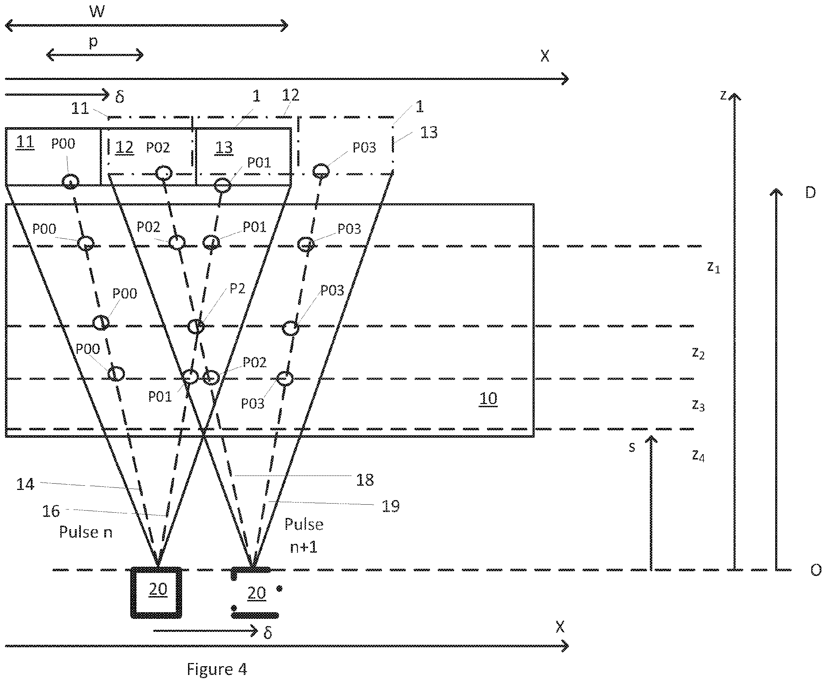

As illustrated in Figure 4, the inspection data is generated as a result of scanning cargo 10 using a matrix 1 comprising a plurality N of at least two rows of detectors and a source 20 of the plurality M of successive pulses. In Figure 4, N=3 and the rows are referenced as 11, 12 and 13. Other numbers N of rows are also envisaged. A pitch p is defined by a distance between a centre of two successive rows or by a width of the matrix 1 divided by N. The matrix 1 is located at a distance D from the source 20.

The pitch p may be a constant in the matrix 1 or may be non-constant in the matrix 1. For example widths of a first row of the matrix 1 and of a last row of the matrix 1 may be larger than width of one or more central rows of the matrix 1. It should be understood that in such a case, the matrix 1 would have several pitch $p_1$, $p_2$,...,$p_N$. The developments below would still apply.

The scanning of the cargo 10 comprises mutually displacing the cargo 10 and the matrix 1 with a mutual scanning displacement. In Figure 4 shows the matrix 1 and the source 20 moving and the cargo 10 not moving during the mutual scanning displacement. The scanning also comprise detecting, with the matrix 1, radiation corresponding to the plurality M of successive X-ray pulses irradiating the cargo 10, during the mutual scanning displacement.

As illustrated in Figure 4, in the inspection data, radiation corresponding to the plurality M of successive pulses irradiating the cargo is arranged in a first order at a level corresponding to the plurality N of rows 11, 12 and 13 of detectors of the matrix 1, in a direction X corresponding to the mutual scanning displacement.

In Figure 4, the level corresponding to the plurality N of rows 11, 12 and 13 of detectors corresponds substantially to Z=D.

In Figure 4, points P00 correspond to radiation direction 14 for pulse n, points P01 correspond to radiation direction 16 for pulse n, points P02 correspond to radiation direction 18 for pulse n+1, and points P03 correspond to radiation direction 19 for pulse n+1.

For example in Figure 4, a radiation incident on the row 11 corresponding to the $n^{th}$ pulse along direction 14 corresponds to a point P00 and another radiation incident on row 13 along direction 16 corresponds to a point P01. Between the pulse n and the pulse (n+1) the matrix 1 and the source 20 move by a distance $\delta$ during the mutual scanning displacement (the cargo 10 is shown as not moving on Figure 4). The radiation incident on row 11 corresponding to the $(n+1)^{th}$ pulse along direction 18 now corresponds to a point P02 and the radiation incident on row 13 along direction 19 corresponds to a point P03. The directions 14 and 18 correspond to the same direction, just translated between pulses n and n+1. The directions 16 and 19 correspond to the same direction, just translated between pulses n and n+1.

Therefore in the example of Figure 4, the radiation corresponding to the plurality M of successive pulses irradiating the cargo (here M=2) is arranged in the following first order at the level corresponding to the plurality N of rows 11, 12 and 13 of detectors, in a direction of increasing X: P00, P02, P01 and P03.

In the example of Figure 4, for covering the whole cargo 10, $\delta$ must be such that:

$$\delta \leq \frac{W.s}{D}$$

with W being a total width of the matrix 1.

Depending on the situation, the displacement $\delta$ may vary from one acquisition to another. In other words, $\delta$ between two pulses may not be a constant during the mutual scanning displacement. Alternatively or additionally, the displacement $\delta$ between two pulses may be a constant during the mutual scanning displacement. For example, in a pass thru mode and at constant source frequency, the displacement $\delta$ may depend on the speed of the cargo passing through the scanner. The displacement $\delta$ is equal to the instantaneous speed of the cargo divided by the frequency of the source. In a mobile mode, the displacement $\delta$ is constant and may be equal to the speed of the scanner divided by the source frequency.

Determining, at S1, the one or more successive reconstruction zones for the inspection data is performed as follows.

The one or more successive reconstruction zones enable selection of one or more reconstruction planes at S2, as explained below.

The one or more reconstruction zones determined at S1 are defined by the experimental conditions (such as the instantaneous speed, the number of matrix rows, the size of the detectors, etc.).

After the one or more reconstruction zones are determined at S1, the one or more reconstruction planes may be selected at S2.

In some examples, one reconstruction plane may be selected for each reconstruction zone. The selected reconstruction plane is located within the determined reconstruction zone. The reconstruction plane may be selected as one of the planes delimiting the reconstruction zone (e.g. the plane closer to the source, but not necessarily). The reconstruction plane may also be selected as being located in the middle of the reconstruction zone, as a non-limiting example.

In some examples, a reconstruction plane may not be selected for each reconstruction zone (e.g. the number of selected reconstruction planes may be lower than the number of determined reconstruction planes), in order to reduce the calculations.

A reconstruction plane enables repositioning acquired data (the data including e.g. the points P01 and P02) at the correct location in the cargo 10. As illustrated in Figure 4, when acquiring data with the matrix 1, the points e.g. P02 and P01 are in the first order, side by side, along the direction X corresponding to the mutual displacement. Each data corresponds to a detector of the matrix and to a pulse. For example P02 corresponds to detector 11 for pulse (n+1) and P01 corresponds to detector 13 for pulse n. When a plane is chosen for reconstruction of the data, the data are back-projected following a projection lines (e.g. dash lines in Figure 4) on a plane at a distance z from the source 20, on virtual pixels whose sizes are the detectors' size divided by the corresponding magnification factor z/D. In Figure 4, point P2, at a distance $z=z_2$, contributes to the two points P02 and P01 at the level z=D (acquired data). The reconstructions of the points P01 and P02 in the plane $z_2$ are thus matching in P2. The plane $z=z_2$ is a correct reconstruction plane. The object P2 corresponding to the data P01 and P02 is located in the plane $z=z_2$, as the back-projection of P01 and P02 converges in P2 at $z=z_2$.

On the contrary, if the data P01 and P02 are reconstructed in a plane $z=z_1$, the two distinct points P01 and P02 are obtained. The plane $z=z_1$ is thus not a correct plane of reconstruction, because the back-projection of P01 and P02 does not converge. The plane z=D is also not a correct reconstruction plane for P01 and P02.

In some examples, each reconstruction zone corresponds to a range of distances from the source 20 in which radiation corresponding to the plurality M of successive pulses irradiating the cargo 10 is arranged in an order which is different from the first order and different from that of another reconstruction zone of the one or more successive reconstruction zones.

Therefore, the one or more reconstruction zones are zones delimited by planes where the ordering of the data changes.

As already explained, in the simple example of Figure 4 the first order (at z=D) is P00, P02, P001 and P03. At $z=z_1$, the order is also P00, P02, P001 and P03. At $z=z_2$, the order changes to P00, P2 and P03. A first reconstruction zone thus corresponds to distances z such that:

$$z_2 \leq z < D.$$

In the simple example of Figure 4, at $z=z_3$, the order is P00, P01, P02 and P03. A second reconstruction zone may thus correspond to distances z such that:

$$z_4 \leq z < z_2.$$

As can be seen on Figure 4, at $z=z_4$ the order is still P00, P01, P02 and P03, and $z_4$ is not a plane for which the order is changing. The plane $z=z_4$ may correspond e.g. to a plane near a face of the cargo 10 located near the source 20. The second reconstruction zone is larger than the range defined by z such that $z_4 \leq z < z_2$.

In a more detailed example, a centre of each row (each row being numbered from 0 to N-1) is back-projected in a plane at a distance z (with z being between D and 0), for each pulse.

In a first example, the mutual scanning displacement $\delta$ between two pulses is not a constant during the mutual scanning displacement. In such an example, determining the one or more successive reconstruction zones for the inspection data is based on an ordered sequence of radiation position X(k,i,z), along the direction of the mutual scanning displacement. The ordered sequence of radiation position X(k,i,z) for an $i^{th}$ row of detectors and a distance z from the source, for the pulse number k, is such that:

$$X(k, i, z) = k.\delta(k) + i.p.\frac{z}{D} + \frac{p}{2}.(1 - \frac{z}{D})$$

The last term $\frac{p}{2}.(1 - \frac{z}{D})$ is a mere translation shift, and is therefore not a critical parameter, so it may be omitted. The determination of the different reconstruction zones is done by determining the different values of z for which the order of the X(k,i,z) is changing.

In some examples, the method 100 may comprise, at S1:

determining X(k,i,z) with k varying from 1 to M, iteratively from z being equal to D to a predetermined distance out of a scanning zone, each iteration being performed using an iterative predetermined decrement d; and
for each iteration:

ordering the determined X(k,i,z), and

determining whether the ordering is different from an ordering in a previous iteration, and

if the ordering is different from an ordering in a previous iteration, determining a corresponding distance from the source as a boundary of a reconstruction zone.

The determining may thus comprise the following steps.

Step 1:

calculation of the X(k, i, z) for z equal to D and with k varying from 1 to the total number M of pulses;
ordering of the X(k, i, D) from the lowest value to the highest value. The ordering is a sequence of (k,i) pairs such as, with M the total number of data equal to a product of the number M of pulses by the number of rows:

$$0 < X(k_1, l_1, D) < X(k_2, l_2, D) < X(k_3, l_3, D) < \ldots < X(k_M, l_M, D);$$

and
store the ordering.

Step 2:

calculation of the X(k, i, z) for a new z, by decreasing z of a value d such that:

$$z = D - d;$$

and
ordering.

If the ordering is the same as the ordering stored at step 1, do not change the stored ordering.

If the ordering is not the same as the ordering stored at step 1, D-d is defining a boundary of a new reconstruction zone, store the new ordering and D-d.

Step 3:

calculation of the X(k, i, z) for z, by decreasing z of a value d such that:

$$z = z - d;$$

and
ordering.

If the order is the same as the ordering at the previous step, do not change the stored ordering.

If the ordering is not the same as the ordering stored at the previous, z-d is defining a boundary of a new reconstruction zone. Store the new ordering and z-d.

Step 4:
iteratively perform step 3 up to a value of z out of the scanning zone (z=D/2 for a mobile scanner, for example).

The above steps do not enable finding exact values of z for which the ordering is changing, but if the value of d is small enough (10 cm, for example) the approximation is good enough.

The sequence of z ($z_0$, $z_1$,..., $z_p$) for which a change in the ordering has been detected is such that:

$$D > z_0 > z_1 > \ldots > z_p > z_{min}.$$

with $z_{min}$ a scanning area plane which is closest to the source.

The planes for which the ordering is changing are only known with an accuracy of d and the output of the above steps is a set of depth zones and of ordered sequence of position along the scanning direction. The reconstructions zones are thus as follows:

$$[z_{min}, z_p], [z_p + d, z_{p-1}], \ldots, [z_1 + d, z_0], [z_0 + d, D].$$

In a second example, the reconstructions zones may be thus as follows, as explained in greater detail below:

$$[Z_{mijn}, z_p] , \ldots, ]z_1, z_0], ]z_0, D].$$

In the second example, the mutual scanning displacement $\delta$ between two pulses is a constant during the mutual scanning displacement. In such an example, determining the one or more successive reconstruction zones for the inspection data is based on an ordered sequence of radiation position X(k,i,z), along the direction of the mutual scanning displacement. The ordered sequence of radiation position X(k,i,z) for an $i^{th}$ row of detectors, for the pulse number k, for a plane z, X(k,i,z) is such that:

$$X(k, i, z) = k.\delta + i.p.\frac{z}{D}.$$

With e.g. N=4 ($0 \leq i \leq 3$), the above positions are a group of four periodic sets of positions, whose common period is:

$$k.\delta, k.\delta + p.\frac{z}{D}, k.\delta + 2.p.\frac{z}{D}, k.\delta + 3.p.\frac{z}{D}.$$

In the interval $[k.\delta, (k+1).\delta[$, there are four data, the first data coming from the first row of detector (i=0). The position of the other three rows in the interval may be determined as follows.

The term $p.\frac{z}{D}$ may be such that:

$$p.\frac{z}{D} = m(z).\delta + r(z) ,$$

with m being integer and r(z) belonging to the interval $[0, \delta[$. Then it follows that:

$$X(k, i, z) = \big(k + i.m(z)\big).\delta + i.r(z)$$

such that m(z).$\delta$+r(z)=p.(z/D), p being a pitch between the plurality N of at least two rows of detectors.

To determine the order of the rows between $[k.\delta, (k+1).\delta[$, the values of $i.r(z)$ may be ordered. Several cases may be possible. Table 1 below shows a list of the ordering of the positions in the interval $[k.\delta, (k+1).\delta[$ as a function of the values of r(z):

Table 1

| | Position 1 | Position 2 | Position 3 | Position 4 |
|---|---|---|---|---|
| **$0 \leq r(z) < \delta/3$** | row: i = 0<br>Pulse number: k<br><br>Position: k.$\delta$ | row: i=1<br>Pulse number: k-m(z)<br><br>Position: k.$\delta$ + r(z) | row: i=2<br>Pulse number: k-2.m(z)<br><br>Position: k.$\delta$ + 2.r(z) | row: i=3<br>Pulse number: k-3.m(z)<br><br>Position: k.$\delta$ + 3.r(z) |
| **$\delta/3 < r(z) < \delta/2$** | row: i = 0<br>Pulse number: k<br><br>Position: k.$\delta$ | row: i=3<br>Pulse number: k-3.m(z)-1<br><br>Position: k.$\delta$ + 3.r(z)-1 | row: i=1<br>Pulse number: k-m(z)<br><br>Position: k.$\delta$ + r(z) | row: i=2<br>Pulse number: k-2.m(z)<br><br>Position: k.$\delta$ + 2.r(z) |
| **$\delta/2 < r(z) < 2.\delta/3$** | row: i = 0<br>Pulse number: k<br><br>Position: k.$\delta$ | row: i=2<br>Pulse number: k-2.m(z)-1<br><br>Position: k.$\delta$ + 2r(z) -1 | row: i=1<br>Pulse number: k-m(z)<br><br>Position: k.$\delta$ + r(z) | row: i=3<br>Pulse number: k-3.m(z)-1<br><br>Position: k.$\delta$ + 3.r(z)-1 |
| **$2.\delta/3 < r(z) < 1$** | row: i = 0<br>Pulse number: k<br><br>Position: k.$\delta$ | row: i=3<br>Pulse number: k-3.m(z)-2<br><br>Position: k.$\delta$ + 3.r(z)-2 | row: i= 2<br>Pulse number: k-2.m(z)-1<br><br>Position: k.$\delta$ + 2.r(z) -1 | row: i=1<br>Pulse number: k-m(z)<br><br>Position: k.$\delta$ + r(z) |

For r(z), the interval $[0, \delta[$ may be split in four intervals, in which the positioning is different, as explained below:

- between 0 and $\delta/3$, the order is normal with: first row (i=0), second row (i=1), third row (i=2) and fourth row (i=3). Nevertheless, the data corresponding to the second, third and fourth rows are acquired for previous X-ray pulses when $m(z) \neq 0$;
- between $\delta/3$ and 5/2, the order is different with: first row (i=0), fourth row (i=3), second row (i=1) and third row (i=2). The pulse number for the fourth row has decreased by one;
- between $\delta/2$ and $2.\delta/3$, the order is: first row (i=0), third row (i=2), second row (i=1) and fourth row (i=3); and
- between $2.\delta/3$ and 1, the order is: first row (i=0), fourth row (i=3), third row (i=2) and first row (i=1).

For a range of z such as r(z) is in the above intervals, the reconstruction may use the rows in the same order as above, and a sequence of pixels based on raw data may be the same as above. Therefore for any object of the cargo placed in the range of such z, the reconstruction will be exact. For objects of the cargo placed closer to or further from the source, artefacts or spread out edges appear in the image due to the effect of the reconstruction zones as explained above. The artefacts or the spread out edges are caused by the mis-ordering of the data when the actual z of the reconstructed object in the cargo is not corresponding to the reconstruction plane.

A number of zones generating different orderings may be determined. The number of zones may be calculated first by estimating initial values of m and r for z=D. After the initial values are estimated, values of z (lower than D) for which the ordering between the rows is changing may be calculated.

For an example with N=4 in a mobile mode with an source frequency of 200Hz, with D being substantially 700 cm and p being 5 mm, $\delta$ is 2 mm (for a speed of 40 cm/s), and

$$p = 2.\delta + \delta/2.$$

Therefore m(D) is 2 and r(D) is $\delta/2$. In this particular case, the plane of the detectors can be considered as a reconstruction plane because r(D) is corresponding to a boundary of one of the mentioned intervals for r. A next interesting plane may be a plane $z_1$ for which $m(z_1)$ is still 2 but for which $r(z_1)$ is $\delta/3$:

$$p.\frac{z_1}{D} = 2.\delta + \frac{\delta}{3} \Rightarrow \frac{z_1}{D} = \frac{7}{3}.\frac{\delta}{p}$$

In this particular case (p=5, $\delta$=2), and $\frac{z_1}{D} = \frac{14}{15}$, hence 653.3 cm. By proceeding in the same way with the other boundaries, the following plans may be determined as listed in Table 2.

Table 2

| m | r | z |
|---|---|---|
| 2 | $\delta/3$ | 653.3 cm |
| | 0 | 560 cm |
| 1 | $2.\delta/3$ | 466.6 cm |
| | $\delta/2$ | 420 cm |
| | $\delta/3$ | 373.3 cm |
| | 0 | 280 cm |
| 0 | $2.\delta/3$ | 186.6 cm |
| | $\delta/2$ | 140 cm |
| | $\delta/3$ | 93.3 cm |

In the above example, nine possible plans where the ordering changes are determined. The four plans which are the closest to the source may not be considered, because the cargo may not be that close to the source. Five possible plans where the ordering changes thus remain, and, because the last plane (z=373.3) does not correspond to a face of the cargo closer to the matrix, it means that the depth information may be used to discriminate the cargo in six different reconstruction zones, in a standard mobile mode with four rows with p equal to five millimetres.

The six reconstruction zones are as follows:

zone 1: 653.3<z≤700 (700=D)
zone 2: 560<z≤653.3
zone 3: 466.6<z≤560
zone 4: 420<z≤466.6
zone 5: 373.3<z≤420
zone 6: $Z_{min}$<z≤373.3

In a case of pass-thru mode, the number of possible plans may be reduced. In a standard case, with a truck speed of 5 km/h and a source frequency of 200 Hz, the value of δ is around 7 mm, which gives m(D)=0 and r(D)~0,71. The only possible planes are corresponding to r(z) equal to 2.δ/3, δ/2 and δ/3, which are, in this case, respectively 648.1 cm, 486.1 cm and 324.1 cm. Only the two first one are located in the cargo (324.1 cm is too close to the source) and the depth information will be reduced to two planes for which the order is changing. There are therefore three reconstruction zones as follows:

zone 1: z> 648.1 cm
zone 2 : 486.1<z≤648.1 cm
zone 3 : s<z≤486.1 cm

In some examples, for higher frequencies, such as 400 Hz or 600 HZ, at least three planes for which the ordering changes may be determined.

As already stated, the method 100 may comprise, selecting at S2 one or more reconstruction planes, based on the determined one or more reconstruction zones.

As already stated, the method 100 may also comprise, for each selected reconstruction plane, generating, at S3, the intermediate image of the cargo.

For a number P of planes where the ordering changes, there may be P+1 reconstruction zones (except when the last plane corresponds to a face of the cargo). If the number of selected planes of reconstruction is equal to the number of zones of reconstruction, a number P+1 of intermediate images may be generated (e.g. reconstructed), the intermediate images corresponding to the P+1 reconstruction planes selected at S2.

The intermediate images may be generated based on the reconstruction plane as follows (in a non-limiting example where it is decided to select one reconstruction plane for each reconstruction zone).

For each of the determined reconstruction zones, a value of z belonging to the corresponding reconstruction zone is selected at S2, such as in the middle or at the boundaries of the reconstruction zone as explained above.

Let $z'_0, z'_1,..., z'_p$ be a sequence such that:

$$(z'_I \in [z_I+d, z_{I-1}]).$$

For zone number I, the ordered sequence of positions is such that:

$$0<X(k_{I1},l_{I1},z'_I)<X(k_{I2},l_{I2}, z'_I)<X(k_{I3},l_{I3},z'_I)<...<X(k_{IM},l_{IM},z'_I).$$

M is the total number of data, product of the number of pulses by the number of rows.

At S3, let S(k,l,o) be data acquired par a detector located at a line o of the matrix 1 and the row I during the pulse k, the line o of the reconstructed image for the zone number I will be the sequence:

$$S(k_{I1},l_{I1},o), \ S(k_{I2},l_{I2},o), \ S(k_{I3},l_{I3},o),...,S(k_{IM},l_{IM},o).$$

In the reconstruction, the sequence of data is not depending on the choice of $z'_I$. However $X(k_{IM},l_{IM},z'_I)$ may be calculated if required.

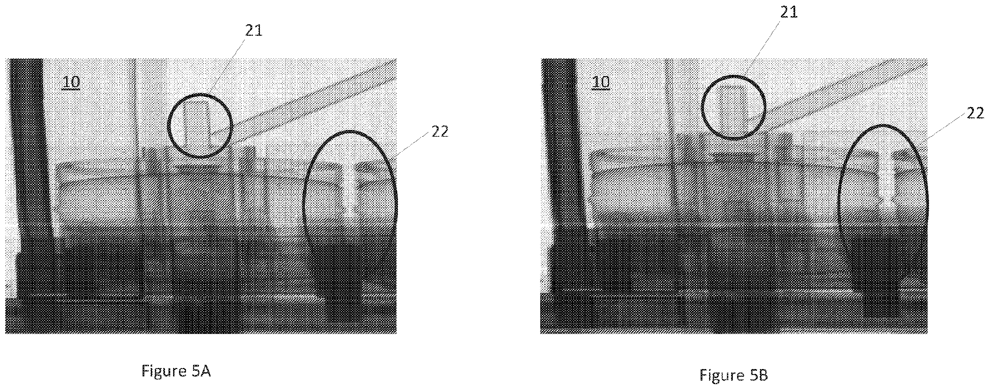

As illustrated in Figure 5A, reconstruction of an object in an area 21 located at a distance z=3.9m from the source, using a reconstruction plane located at 3.9m from the source, generates an intermediate image without an improper gradient in a direction corresponding to the mutual scanning displacement. As illustrated in Figure 5A, reconstruction of an object in an area 22 located at a distance z=6.5m from the source, using the reconstruction plane located at z=3.9m from the source, generates the intermediate image with a gradient (e.g. artefacts or spread out edges) in a direction corresponding to the mutual scanning displacement.

As illustrated in Figure 5B, reconstruction of the object in the area 21 located at a distance z=3.9m from the source, using a reconstruction plane located at 6.5m from the source, generates an intermediate image with a gradient (e.g. artefacts or spread out edges) in a direction corresponding to the mutual scanning displacement. As illustrated in Figure 5B, reconstruction of the object in the area 22 located at a distance z=6.5m from the source, using the reconstruction plane located at 6.5m from the source, generates the intermediate image without an improper gradient in a direction corresponding to the mutual scanning displacement.

Therefore as illustrated in Figures 5A and 5B, when the reconstruction zone does not correspond to the object location in the cargo, objects with relatively high horizontal gradient show artefacts or have spread out edges in the image.

As already stated, the method 100 may comprise generating, at S4, an average image by averaging all of the generated intermediate images.

On the generated average image, the selecting, at S5, of the one or more pixels having a horizontal gradient (i.e. in the direction corresponding to the mutual scanning displacement) which is greater in absolute value than a predetermined threshold enable identification of vertical or oblique edges.

The absolute value of the horizontal gradient can be calculated for example by a standard formula of horizontal variation along direction x of pixel (i,j) such as:

$$\left| \nabla_{x,i,j} \right| = \left| I_{i+1,j} - I_{i-1,j} \right| / 2$$

$I_{i,j}$ being the intensity of the pixel (i,j) with i being the horizontal coordinate (i.e. in the scanning direction).

Alternatively or additionally, the absolute value of the horizontal grandient can also be calculated based on a Sobel mask.

The threshold Th can be equal to a parameter A having constant value. To take into account the noise dependency of the signal, the threshold Th can also be implemented as a function of the pixel average in a neighbourhood such that:

$$Th_{i,j} = A.f\left( \sum_{neighbourhood} I_{i',j'} \right)$$

In such a case the threshold Th varies from one pixel to another.

The predetermined parameter A may be chosen by a user of an inspection system implementing the method according to any aspects of the disclosure.

As already stated, the method 100 may comprise, for each one of the pixels selected at S5, extracting, at S6, a neighbourhood of the selected pixel from each generated intermediate image. For each of the pixels selected at S5, S6 enables to extract P+1 neighbourhoods (e.g. thumbnails). In some examples the neighbourhood may correspond to an 8-connected neighbourhood.

As already stated, the method 100 may comprise, determining, at S7, in the extracted neighbourhoods, the neighbourhood minimizing a criterion compared with the other extracted neighbourhoods. The minimal criterion may be associated for examples with sharper edges (e.g. fewer artefacts) in the determined neighbourhood. The determined neighbourhood may thus have fewer artefacts than the other extracted neighbourhoods.

As already discussed with reference to Figures 5A and 5B, horizontal gradients (e.g. corresponding to artefacts) appear in vertical edges of the objects and look like discontinuous lines when the objects are reconstructed with a reconstruction plane which is different from the reconstruction plane to which the objects belong. Based on this observation, the criterion to minimize may be an energy E which may be determined as follows. The energy E comprises two terms: a first term being a horizontal gradient, squared, and a second term being a penalization function as defined as followed. The neighbourhood having the lowest energy E is selected.

In some embodiments, determining at S7 the neighbourhood having the minimal criterion may comprise:

for each value $I_{ij,p}$ of the $p^{th}$ generated intermediate image Ip, in coordinates (i,j) corresponding to one of the selected pixels, and for all of the generated intermediate image Ip, determining the energy E such that:

$$E\left( I_{ij,p} \right) = \left( \nabla_x I \right)^2_{ij,p} + g(I_{ij,p})$$

with the first term being a gradient along the direction x of the mutual scanning displacement, squared, and a second term being a penalization function g configured to penalize, in the minimizing, neighbourhoods with different gray levels.

In some examples, the determining at S7 may also comprise selecting the neighbourhood associated with the generated intermediate image Ip having a minimal determined energy.

In some examples, the penalization function g is a Geman-McClure function defined by:

$$g(I_{ij,p}) = \sum_{k \in N_{ij}^6} \rho(r_k, \sigma(r_k))$$

with k is a value used to index the pixels of the neighbourhood of the pixel (i,j). In the $N_{ij}^6$ neighbourhood, they correspond to the pixels (i-1,j+1),(i-1,j),(i-1,)-1),(i+1,j+1),(i+1,j),(i+1,j-1), k is varying from 1 to 6, with $\rho(r,\sigma) = \frac{r^2}{r^2+\sigma^2}$, $r_k = \left\{ I_k - I_{ij} \mid k \in N_{ij}^6 \right\}$, $I_k$ being values of the images in the neighbourhood $N_{ij}^6$, $I_{ij}$ being the value of the image for the current pixel (i,j), and
$\sigma(r_k)$ is the standard deviation of the value of $r_k$ on the neighbourhood

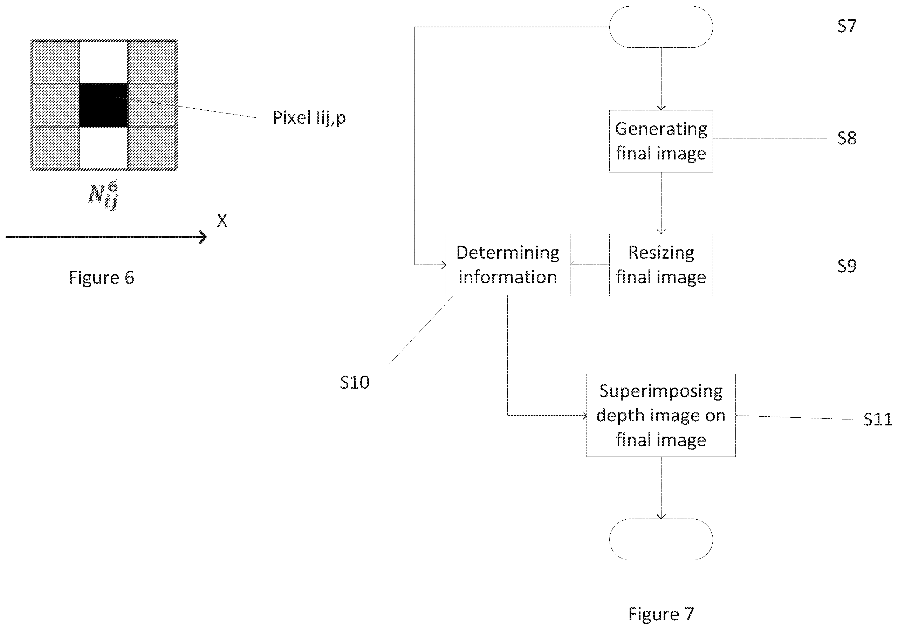

As illustrated in Figure 6, N6ij a neighbourhood having white pixels above and below the pixel of value $I_{ij,p}$ in a direction perpendicular to the direction of the mutual scanning displacement x, and having grey pixels elsewhere in the neighbourhood. $N_{ij}^6$ is the neighborhood of the pixel located in (i,j), indexed by k from 1 to 6, and whose constitutive pixels are the three closest pixels to (i,j) located in the column before and the three closest pixels to (i,j) located in the column after.

The function g penalizes neighbourhoods with different gray levels. If gray lines in Figure 6 are discontinuous lines due to stereoscopic effect, $r_k$ will have high values and g will also have high values, which will penalize E. E will not have high values if the neighbourhood is a homogeneous area.

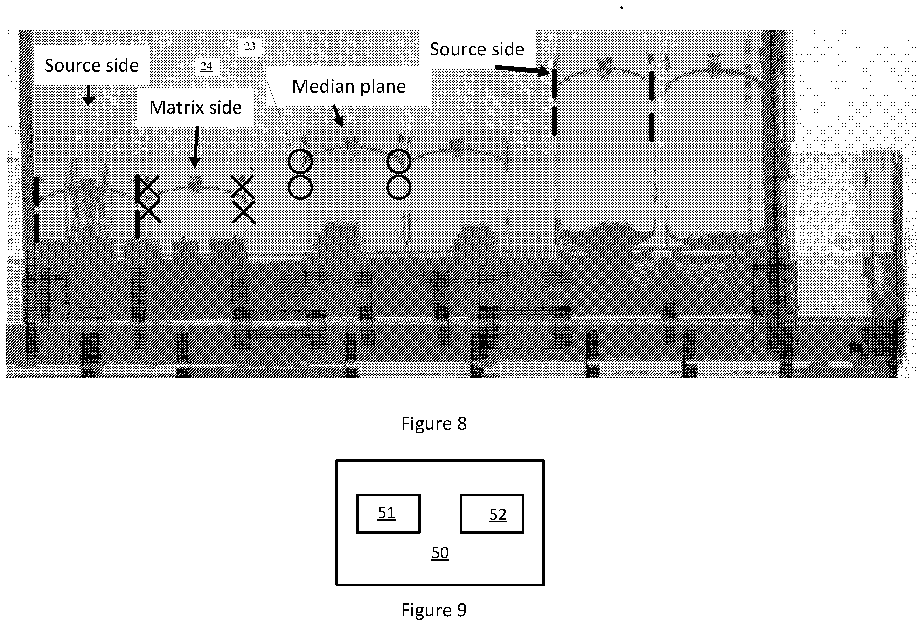

In a first example, as illustrated in Figure 7, the method 100 may further comprise generating, at S8, a final image of the cargo. The final image may correspond to a corrected average image. An example final image 24 is illustrated in Figure 8.

In some examples, generating at S8 the final image comprising pixels may comprise:

assigning, for each pixel of the final image corresponding to one of the selected pixels, a value of the pixel in the generated intermediate image corresponding to the neighbourhood determined at S7; and
assigning, for each other pixel of the final image, a value of the pixel in the average image determined at S4 or in the generated intermediate image corresponding to a reconstruction zone closest to the matrix determined at S3.

The method 100 may comprise resizing, at S9, the generated final image, in the direction corresponding to the mutual scanning displacement and/or perpendicularly to the direction corresponding to the mutual scanning displacement, to obtain a width on height ratio corresponding to a median plane of the cargo, the median plane being substantially parallel to the matrix.

Alternatively or additionally, in a second example, as illustrated in Figure 7, the method 100 may further comprise, for each pixel corresponding to one of the pixels selected at S5, determining, at S10, information relating to the determined reconstruction zone corresponding to the determined neighbourhood having the fewer artefacts.

As illustrated in Figure 7, the determining at S10 may be performed after the step S7. In other words, the pixel corresponding to one of the selected pixels may comprise at least one pixel of the generated average image. Alternatively or additionally, the determining at S10 may be performed after the step S8 or the step S9. In other words, the pixel corresponding to one of the selected pixels may comprise at least one pixel of the final image of the cargo.

In some examples, the determined information relating to the determined reconstruction zone comprises:

an ordinal number corresponding to a relative position of the determined reconstruction zone in the one or more successive reconstruction zones and/or a depth zone, each pixel not corresponding to one of the selected pixels not being associated with an ordinal number and/or a depth zone; and/or
at least one distance from the source corresponding to at least one boundary of the range of distances associated with the determined reconstruction zone in the one or more successive reconstruction zones.

In some examples, determining at S10 the information may further comprise:

predetermining a number of possible depth zones, the number of possible depth zones being equal to or smaller than a number of the determined one or more successive reconstruction zones;
merging the determined one or more successive reconstruction zones into one or more depths zones, a number of the one or more depths zones corresponding to the number of possible depth zones;
assigning a merged depth zone to each pixel corresponding to one of the selected pixels, based on the determined information; and

generating a depth image based on the assigning, each pixel not corresponding to one of the selected pixels not being assigned to a merged depth zone.

In some examples the number of possible depth zones may be chosen by the user of the inspection system implementing the method according to any aspects of the disclosure, and may be equal to e.g. three (corresponding to e.g. a detector side, a middle of the cargo or median plane, and/or an source side).

In the merging, for example, if we have the following reconstruction zones:

$$[z_{mijn}, z_p], [z_p+d, z_{p-1}], \dots, [z_1+d, z_0], [z_0+d, D]$$

and if the predetermined number of possible depth zones is three, the merged depth zones may be as follows:

$$[z_{mijn}, z_{p1}], [z_{p1}+d, z_{p2}], [z_{p2}+d, D]$$

with $z_{l1}$ and $z_{l2}$ depth values from the sequence $z_0, z_1, \dots, z_p$.

In the example of Figure 7, the method 100 may further comprise superimposing, at S11, the depth image generated at S10 on the final image of the cargo generated at S8 and/or S9.

Figure 8 illustrates a depth image 23 superimposed on the final image 11 of the cargo.

In the example of Figure 8, the predetermined number of possible depth zones is three, and the merged depth zones correspond to "source side", "median plane" and "matrix side". The depth image 23 may comprise visual markers to indicate the depth information. In the example of Figure 8, the depth image 23 may comprise straight lines to indicate that the corresponding object is located on the source side in the cargo, circles to indicate that the corresponding object is located on the median plane in the cargo, and crosses to indicate that the corresponding object is located on the source side in the cargo. Other visual markers may be envisaged, such as colours (e.g. red for source side, green for median plane and blue for matrix side, as non-limiting examples).

The generated depth image may be noisy, because e.g. of spurious variations of depth from one pixel to another pixel in the neighbourhoods. The method may thus further comprise denoising the generated depth image by forcing adjacent pixels to belong to a common depth zone. In some examples, the forcing may be based on the following constraint: *two pixels vertically adjacent cannot belong to two different reconstruction planes.*

As illustrated in Figure 9, the disclosure also relates to a system 50 comprising a processor 51 and a memory 52 storing instructions which, when executed by the processor, enable the processor to perform a method according to any aspect of the present disclosure.

In some examples the source is configured to irradiate the cargo with at least two different levels of energy for material discrimination. In such examples, the method of any aspect of the disclosure may be performed for each of the at least two different levels of energy.

In the examples above, the inspection radiation source may comprise an X-ray generator. The energy of the X-rays may be comprised between 100keV and 15MeV, and the dose rate may be comprised between 2mGy and 20Gy (Gray) per minute at one meter from the source. The maximum X-ray energy of the X-ray source may be e.g., between 100keV and 9.0MeV, typically e.g., 2MeV, 3.5MeV, 4MeV, or 6MeV, for a steel penetration capacity e.g., between 40mm to 400mm, typically e.g., 300mm (12in). The dose may be e.g., between 20mGy and 120mGy. In other examples, the maximum x-ray energy of the X-ray source may be e.g., between 4MeV and 10MeV, typically e.g., 9MeV, for a steel penetration capacity e.g., between 300mm to 450mm, typically e.g., 410mm (16.1in). In some examples, the dose may be 17Gy.

An inspection system implementing the method may further comprise other types of detectors, such as optional gamma and/or neutrons detectors, e.g., adapted to detect the presence of radioactive gamma and/or neutrons emitting materials within the cargo 10, e.g., simultaneously to the X-ray inspection.

**Claims**

1. A computer-implemented method for processing inspection data associated with cargo irradiated by a plurality M of successive pulses of X-rays, comprising:

    obtaining the inspection data,

        the inspection data being generated as a result of scanning the cargo using a matrix comprising a plurality N of at least two rows of detectors, and a source of the plurality M of successive pulses, the matrix being located at a distance D from the source, the scanning comprising:

mutually displacing the cargo and the matrix with a mutual scanning displacement, and
detecting, with the matrix, radiation corresponding to the plurality M of successive X-ray pulses irradiating the cargo, during the mutual scanning displacement,

wherein, in the inspection data, radiation corresponding to the plurality M of successive pulses irradiating the cargo and detected by the plurality N of at least two rows of detectors is arranged in a first order corresponding to a level of the matrix, in a direction corresponding to the mutual scanning displacement;

determining one or more successive reconstruction zones for the inspection data, wherein each reconstruction zone corresponds to a range of distances from the source in which radiation corresponding to the plurality M of successive pulses irradiating the cargo is arranged in an order which is different from the first order and different from that of another reconstruction zone of the one or more successive reconstruction zones;
selecting one or more reconstruction planes, based on the determined one or more reconstruction zones;
for each selected reconstruction plane, generating an intermediate image of the cargo;
generating an average image by averaging all of the generated intermediate images;
on the generated average image, selecting one or more pixels having a gradient, in a direction corresponding to the mutual scanning displacement, greater in absolute value than a predetermined threshold; and
for each one of the selected pixels:

extracting a neighbourhood of the selected pixel from each generated intermediate image, and
determining, in the extracted neighbourhoods, the neighbourhood minimizing a criterion compared with the other extracted neighbourhoods.

2. The method of the previous claim, further comprising generating a final image of the cargo corresponding to a corrected average image, the final image comprising pixels and generating the final image comprising:

assigning, for each pixel of the final image corresponding to one of the selected pixels, a value of the pixel in the generated intermediate image corresponding to the determined neighbourhood; and
assigning, for each other pixel of the final image, a value of the pixel in the average image or in the generated intermediate image corresponding to a reconstruction zone closest to the matrix.

3. The method of any of the previous claims, further comprising resizing the generated final image, in the direction corresponding to the mutual scanning displacement and/or perpendicularly to the direction corresponding to the mutual scanning displacement, to obtain a width on height ratio corresponding to a median plane of the cargo, the median plane being substantially parallel to the matrix.

4. The method of any of the previous claims, further comprising, for each pixel corresponding to one of the selected pixels:

determining information relating to the determined reconstruction zone corresponding to the determined neighbourhood, optionally wherein determining the information further comprises:
predetermining a number of possible depth zones, the number of possible depth zones being equal to or smaller than a number of the determined one or more successive reconstruction zones;
merging the determined one or more successive reconstruction zones into one or more depths zones, a number of the one or more depths zones corresponding to the number of possible depth zones;
assigning a merged depth zone to each pixel corresponding to one of the selected pixels, based on the determined information; and
generating a depth image based on the assigning, each pixel not corresponding to one of the selected pixels not being assigned to a merged depth zone.

5. The method of the previous claim, further comprising generating a final image of the cargo corresponding to a corrected average image, the final image comprising pixels and generating the final image comprising:

assigning, for each pixel of the final image corresponding to one of the selected pixels, a value of the pixel in the generated intermediate image corresponding to the determined neighbourhood; and

assigning, for each other pixel of the final image, a value of the pixel in the average image or in the generated intermediate image corresponding to a reconstruction zone closest to the matrix, and further comprising:

superimposing the generated depth image on the generated final image of the cargo, optionally further comprising:

denoising the generated depth image by forcing adjacent pixels to belong to a common depth zone, optionally wherein the pixel corresponding to one of the selected pixels comprises:

at least one pixel of the generated average image, and/or
at least one pixel of the final image of the cargo,
optionally wherein the determined information relating to the determined reconstruction zone comprises:
an ordinal number corresponding to a relative position of the determined reconstruction zone in the one or more successive reconstruction zones and/or a depth zone, each pixel not corresponding to one of the selected pixels not being associated with an ordinal number and/or a depth zone; and/or
at least one distance from the source corresponding to at least one boundary of the range of distances associated with the determined reconstruction zone in the one or more successive reconstruction zones.

6. The method of any of the previous claims, wherein a mutual scanning displacement $\delta$ between two pulses is a constant during the mutual scanning displacement, and wherein determining the one or more successive reconstruction zones for the inspection data is based on an ordered sequence of radiation position $X(k,i,z)$, along a direction of the mutual scanning displacement, associated with an $i^{th}$ row of detectors and a distance z from the source, for the pulse number k, such that:

$$X(k,i,z)=(k+i.m(z)).\delta+i.r(z)$$

with m being an integer,

r(z) belonging to the interval $[0,\delta[$, and
such that $m(z).\delta+r(z)=p.(z/D)$, p being a pitch between the plurality N of at least two rows of detectors.

7. The method of any of claims 1 to 6, wherein a mutual scanning displacement $\delta$ between two pulses is not a constant during the mutual scanning displacement, and wherein determining the one or more successive reconstruction zones for the inspection data is based on an ordered sequence of radiation position $X(k,i,z)$, along a direction of the mutual scanning displacement, corresponding to an $i^{th}$ row of detectors and a distance z from the source, for the pulse number k, such that:

$$X(k, i, z) = k.\delta(k) + i.p.\frac{z}{D} + \frac{p}{2}.(1 - \frac{z}{D})$$

with p being a pitch between the plurality N of at least two rows of detectors, optionally further comprising:

determining $X(k,i,z)$ with k varying from 1 to M, iteratively from z being equal to D to a predetermined distance out of a scanning zone, each iteration being performed using an iterative predetermined decrement d; and
for each iteration:

ordering the determined $X(k,i,z)$,
determining whether the ordering is different from an ordering in a previous iteration, and
if the ordering is different from an ordering in a previous iteration, determining a corresponding distance from the matrix as a boundary of a reconstruction zone.

8. The method of any of the previous claims, wherein determining the neighbourhood having the minimal criterion comprises:

for each value $I_{ij,p}$ of the $p^{th}$ generated intermediate image Ip, in coordinates (i,j) corresponding to one of the selected pixels, and for all of the generated intermediate image Ip, determining energy E such that:

$$E\left(I_{ij,p}\right) = \ (\nabla_x I)^2{}_{ij,p} + g(I_{ij,p})$$

with a first term being a gradient along the direction x of the mutual scanning displacement, squared, and a second term being a penalization function g configured to penalize, in the minimizing, neighbourhoods with different gray levels; and
selecting the neighbourhood associated with the generated intermediate image Ip having a minimal determined energy, optionally wherein g is a Geman-McClure function defined by:

$$g\left(I_{ij,p}\right) = \ \sum_{k\in N_{ij}^6} \rho(r_k, \sigma(r_k))$$

with $N_{ij}^6$ being the neighborhood of the pixel located in (i,j), indexed by k from 1 to 6, and whose constitutive pixels are the three closest pixels to (i,j) located in the column before and the three closest pixels to (i,j) located in the column after,

$$\rho(r,\sigma) = \ \frac{r^2}{r^2+\sigma^2},$$

$r_k = \left\{\ I_k - I_{ij}\ \middle|\ k \in N_{ij}^6\right\}$, $I_{ij}$ being the image value at pixel (i,j) and $I_k$ the image values for the pixel in the neighbourhood $N_{ij}^6$, and
$\sigma(r_k)$ is the standard deviation of the value of $r_k$ on the neighbourhood.

9. The method of any of the previous claims, wherein the neighbourhood is an 8-connected neighbourhood.

10. The method of any of the previous claims, wherein the source is configured to irradiate the cargo with at least two different levels of energy for material discrimination, the method for processing the inspection data being performed for each of the at least two different levels of energy.

11. The method of any of the previous claims, wherein the absolute value of the gradient along direction x is such that:

$$\left|\nabla_{x,i,j}\right| = \ \left|I_{i+1,j} - I_{i-1,j}\right|/2$$

with $I_{i,j}$ being an intensity of a pixel (i,j), with i being the coordinate in the direction x corresponding to the mutual scanning displacement, and/or
the absolute value of the gradient is calculated based on a Sobel mask.

12. The method of any of the previous claims, wherein the predetermined threshold is a constant value or varies from one pixel to another pixel.

13. A system comprising:

a processor; and
a memory storing instructions which, when executed by the processor, enable the processor to perform a method for processing inspection data associated with cargo irradiated by a plurality M of successive pulses of X-rays, comprising:
obtaining the inspection data,

the inspection data being generated as a result of scanning the cargo using a matrix comprising a plurality N of at least two rows of detectors, and a source of the plurality M of successive pulses, the matrix being located at a distance D from the source, the scanning comprising:

mutually displacing the cargo and the matrix with a mutual scanning displacement, and
detecting, with the matrix, radiation corresponding to the plurality M of successive X-ray pulses irradiating the cargo, during the mutual scanning displacement,

wherein, in the inspection data, radiation corresponding to the plurality M of successive pulses irradiating the cargo and detected by the plurality N of at least two rows of detectors is arranged in a first order corresponding to a level of the matrix, in a direction corresponding to the mutual scanning displacement;

determining one or more successive reconstruction zones for the inspection data, wherein each reconstruction zone corresponds to a range of distances from the source in which radiation corresponding to the plurality M of successive pulses irradiating the cargo is arranged in an order which is different from the first order and different from that of another reconstruction zone of the one or more successive reconstruction zones;
selecting one or more reconstruction planes, based on the determined one or more reconstruction zones;
for each selected reconstruction plane, generating an intermediate image of the cargo;
generating an average image by averaging all of the generated intermediate images;
on the generated average image, selecting one or more pixels having a gradient, in a direction corresponding to the mutual scanning displacement, greater in absolute value than a predetermined threshold; and
for each one of the selected pixels:

extracting a neighbourhood of the selected pixel from each generated intermediate image, and
determining, in the extracted neighbourhoods, the neighbourhood minimizing a criterion compared with the other extracted neighbourhoods.

14. The system of the preceding claim, wherein the memory stores instructions which, when executed by the processor, enable the processor to perform the method of any one of claims 2 to 12.

15. A computer program product or a computer program comprising instructions which, when executed by a processor, enable the processor to perform the method of any of claims 1 to 12 or to provide the system of claim 13 or 14.

**Patentansprüche**

1. Computerimplementiertes Verfahren zum Verarbeiten von Inspektionsdaten, die mit einer Fracht in Verbindung stehen, die durch eine Vielzahl M aufeinanderfolgender Impulse von Röntgenstrahlen bestrahlt wird, Folgendes umfassend:

Erhalten der Inspektionsdaten,

wobei die Inspektionsdaten im Ergebnis des Scannens der Fracht mit Hilfe einer Matrix generiert werden, welche eine Vielzahl N von mindestens zwei Reihen aus Detektoren und eine Quelle der Vielzahl M aufeinanderfolgender Impulse umfasst, wobei sich die Matrix in einem Abstand D zu der Quelle befindet, wobei das Scannen Folgendes umfasst:

wechselseitiges Verschieben der Fracht und der Matrix mit einer wechselseitigen Scan-Verschiebung, und
Detektieren, mit der Matrix, von Strahlung, die der Vielzahl M aufeinanderfolgender Röntgenstrahl-Impulse, welche die Fracht bestrahlen, entspricht, während der wechselseitigen Scan-Verschiebung,

wobei in den Inspektionsdaten Strahlung, die der Vielzahl M aufeinanderfolgender Impulse, welche die Fracht bestrahlen und durch die Vielzahl N von mindestens zwei Reihen aus Detektoren detektiert werden, in einer ersten Reihenfolge angeordnet sind, die einer Ebene der Matrix in einer Richtung entspricht, die der wechselseitigen Scan-Verschiebung entspricht;

Bestimmen einer oder mehrerer aufeinanderfolgender Rekonstruktionszonen für die Inspektionsdaten, wobei jede Rekonstruktionszone einem Bereich von Abständen zu der Quelle entspricht, in dem Strahlung, die der Vielzahl M aufeinanderfolgender Impulse, welche die Fracht bestrahlen, in einer Reihenfolge angeordnet ist, die sich von der ersten Reihenfolge unterscheidet und sich von der einer anderen Rekonstruktionszone der einen oder mehreren aufeinanderfolgenden Rekonstruktionszonen unterscheidet;

Auswählen einer oder mehrerer Rekonstruktionsebenen, basierend auf der bestimmten einen oder den bestimmten mehreren Rekonstruktionszonen;

für jede ausgewählte Rekonstruktionsebene Generieren eines Zwischenbildes der Fracht;

Generieren eines Durchschnittsbildes durch Mitteln aller generierten Zwischenbilder;

auf dem generierten Durchschnittsbild Auswählen eines oder mehrerer Pixel, die in einer Richtung, die der wechselseitigen Scan-Verschiebung entspricht, einen Gradienten aufweisen, dessen absoluter Wert größer als ein festgelegter Schwellenwert ist; und

für jedes der ausgewählten Pixel:

> Extrahieren einer Nachbarschaft des ausgewählten Pixels von jedem generierten Zwischenbild, und
> Bestimmen, in den extrahierten Nachbarschaften, der Nachbarschaft, die im Vergleich mit den anderen extrahierten Nachbarschaften ein Kriterium minimiert.

2. Verfahren nach dem vorhergehenden Anspruch, ferner das Generieren eines endgültigen Bildes der Fracht umfassend, das einem korrigierten Durchschnittsbild entspricht, wobei das endgültige Bild Pixel umfasst und das Generieren des endgültigen Bildes Folgendes umfasst:

> Zuweisen, für jedes Pixel des endgültigen Bildes, das einem der ausgewählten Pixel entspricht, eines Wertes des Pixels in dem generierten Zwischenbild, der der bestimmten Nachbarschaft entspricht; und
> Zuweisen, für jedes andere Pixel des endgültigen Bildes, eines Wertes des Pixels in dem Durchschnittsbild oder in dem generierten Zwischenbild, der einer Rekonstruktionszone entspricht, die der Matrix am nächsten liegt.

3. Verfahren nach einem der vorhergehenden Ansprüche, ferner das Ändern der Größe des generierten endgültigen Bildes in der Richtung umfassend, die der wechselseitigen Scan-Verschiebung entspricht, und/oder senkrecht zu der Richtung, die der wechselseitigen Scan-Verschiebung entspricht, um ein Verhältnis von Breite zu Höhe zu erhalten, das einer Medianebene der Fracht entspricht, wobei die Medianebene im Wesentlichen parallel zur Matrix liegt.

4. Verfahren nach einem der vorhergehenden Ansprüche, ferner für jedes Pixel, das einem der ausgewählten Pixel entspricht, Folgendes umfassend:

> Bestimmen von Informationen bezüglich der bestimmten Rekonstruktionszone, die der bestimmten Nachbarschaft entspricht, optional wobei das Bestimmen der Informationen Folgendes umfasst:
> Festlegen einer Anzahl möglicher Tiefenzonen, wobei die Anzahl der möglichen Tiefenzonen gleich oder kleiner als eine Anzahl der bestimmten einen oder mehreren aufeinanderfolgenden Rekonstruktionszonen ist;
> Zusammenführen der bestimmten einen oder mehreren aufeinanderfolgenden Rekonstruktionszonen zu einer oder mehreren Tiefenzonen, wobei eine Anzahl der einen oder mehreren Tiefenzonen der Anzahl möglicher Tiefenzonen entspricht;
> Zuweisen einer zusammengeführten Tiefenzonen zu jedem Pixel, das dem einen der ausgewählten Pixel entspricht, basierend auf den bestimmten Informationen; und
> Generieren eines Tiefenbildes basierend auf der Zuweisung, wobei jedes Pixel, das keinem der ausgewählten Pixel entspricht, keiner zusammengeführten Tiefenzone zugewiesen wird.

5. Verfahren nach dem vorhergehenden Anspruch, ferner das Generieren eines endgültigen Bildes der Fracht, das einem korrigierten Durchschnittsbild entspricht, umfassend, wobei das endgültige Bild Pixel umfasst und das Generieren des endgültigen Bildes Folgendes umfasst:

> Zuweisen, für jedes Pixel des endgültigen Bildes, das einem der ausgewählten Pixel entspricht, eines Wertes des Pixels in dem Generieren Zwischenbild, der der bestimmen Nachbarschaft entspricht, und
> Zuweisen, für jedes andere Pixel des endgültigen Bildes, eines Wertes des Pixels in dem Durchschnittsbild oder in dem generierten Zwischenbild, der einer Rekonstruktionszone entspricht, die der Matrix am nächsten liegt, und ferner Folgendes umfassend:
> Überblenden des generierten endgültigen Bildes der Fracht mit dem generierten Tiefenbild, optional ferner Folgendes umfassend:
> Entrauschen des generierten Tiefenbildes durch Erzwingen, das benachbarte Pixel zu einer gemeinsamen Tiefenzone gehören,
> optional wobei das Pixel, das einem der ausgewählten Pixel entspricht, Folgendes umfasst:

mindestens ein Pixel des generierten Durchschnittsbildes und/oder
mindestens ein Pixel des endgültigen Bildes der Fracht,
optional wobei die bestimmten Informationen bezüglich der bestimmten Rekonstruktionszone Folgendes umfassen:

> eine Ordnungszahl, die einer relativen Position der bestimmten Rekonstruktionszone in der einen oder den mehreren aufeinanderfolgenden Rekonstruktionszonen und/oder einer Tiefenzone entspricht, wobei keinem der Pixel, die keinem der ausgewählten Pixel entsprechen, eine Ordnungszahl und/oder eine Tiefenzone zugeordnet wird; und/oder
> mindestens ein Abstand zur Quelle, der mindestens einer Grenze des Bereichs von Abständen entspricht, welcher der bestimmten Rekonstruktionszone in der einen oder den mehreren aufeinanderfolgenden Rekonstruktionszonen zugeordnet ist.

6. Verfahren nach einem der vorhergehenden Ansprüche, wobei eine wechselseitige Scan-Verschiebung $\delta$ zwischen zwei Impulsen während der wechselseitigen Scan-Verschiebung eine Konstante ist und wobei das Bestimmen der einen oder mehreren aufeinanderfolgenden Rekonstruktionszonen für die Inspektionsdaten auf einer in Reihenfolge geordneten Abfolge der Strahlungsposition X(k,i,z) entlang einer Richtung der wechselseitigen Scan-Verschiebung basiert, die einer i. Reihe aus Detektoren und einem Abstand z zu der Quelle für die Impulsanzahl k entspricht, so dass:

$$X(k,i,z)=(k+i.m(z)).\delta+i.r(z)$$

wobei m eine Ganzzahl ist,

> r(z) zu dem Intervall $[0,\delta[$ gehört, und
> so, dass $m(z).\delta+r(z)=p.(z/D)$, wobei p ein Zwischenraum zwischen der Vielzahl N von mindestens zwei Reihen aus Detektoren ist.

7. Verfahren nach einem der Ansprüche 1 bis 6, wobei eine wechselseitige Scan-Verschiebung $\delta$ zwischen zwei Impulsen keine Konstante während der wechselseitigen Scan-Verschiebung ist und wobei das Bestimmen der einen oder mehreren aufeinanderfolgenden Rekonstruktionszonen für die Inspektionsdaten auf einer in Reihenfolge geordneten Abfolge der Strahlungsposition X(k,i,z) entlang einer Richtung der wechselseitigen Scan-Verschiebung basiert, die einer i. Reihe aus Detektoren und einem Abstand z zu der Quelle für die Impulsanzahl k entspricht, so dass:

$$X(k, i, z) = k.\delta(k) + i.p.\frac{z}{D} + \frac{p}{2}.(1 - \frac{z}{D})$$

wobei p ein Zwischenraum zwischen der Vielzahl N von mindestens zwei Reihen aus Detektoren ist, optional ferner Folgendes umfassend:
iteratives Bestimmen von X(k,i,z), wobei k von 1 bis M variiert, ausgehend von z gleich D bis zu einem festgelegen Abstand außerhalb der Scan-Zone, wobei jede Iteration mit Hilfe eines iterativen festgelegten Dekrements d durchgeführt wird; und für jede Wiederholung:

> Ordnen des bestimmten X(k,i,z) in Reihenfolge,
> Bestimmen, ob sich die Reihenfolge von einer Reihenfolge in einer vorherigen Iteration unterscheidet, und wenn sich die Reihenfolge von einer Reihenfolge in einer vorherigen Iteration unterscheidet, Bestimmen eines entsprechenden Abstandes zu der Matrix als eine Grenze einer Rekonstruktionszone.

8. Verfahren nach einem der vorhergehenden Ansprüche, wobei das Bestimmen der Nachbarschaft mit dem Minimalkriterium Folgendes umfasst:

> für jeden Wert $I_{ij,p}$ des p. generierten Zwischenbildes Ip, in Koordinaten (i,j), die einem der ausgewählten Pixel entsprechen, und für alle des erzeugten Zwischenbildes Ip, Bestimmen der Energie E derart, dass:

$$E\left(I_{ij,p}\right) = (\nabla_x I)^2{}_{ij,p} + g(I_{ij,p})$$

wobei ein erster Term ein Gradient entlang der Richtung x der wechselseitigen Scan-Verschiebung zum Quadrat ist, und
ein zweiter Term eine Penalisierungsfunktion g ist, die dafür konfiguriert ist, beim Minimieren Nachbarschaften mit unterschiedlichen Graustufen zu penalisieren; und
Auswählen der Nachbarschaft, die dem generierten Zwischenbild Ip zugeordnet ist, das eine minimale bestimmte Energie aufweist, optional wobei g eine Geman-McClure-Funktion ist, die definiert ist durch:

$$g\left(I_{ij,p}\right) = \sum_{k \in N_{ij}^6} \rho(r_k, \sigma(r_k))$$

wobei $N_{ij}^6$ die Nachbarschaft des Pixels ist, die sich in (i,j) befindet, indiziert durch k von 1 bis 6 und deren konstitutive Pixel die drei nächstliegenden Pixel zu (i,j) sind, die sich in der senkrechten Reihe davor befinden, und die drei nächstliegenden Pixel zu (i,j), die sich in der senkrechten Reihe danach befinden,

$$\rho(r, \sigma) = \frac{r^2}{r^2 + \sigma^2},$$

$r_k = \left\{ I_k - I_{ij} \mid k \in N_{ij}^6 \right\}$ , wobei $I_{ij}$ der Bildwert bei Pixel (i,j) ist und $I_k$ die Bildwerte für das Pixel in der Nachbarschaft $N_{ij}^6$ sind, und $\sigma(r_k)$ die Standardabweichung des Wertes von $r_k$ in der Nachbarschaft ist.

9. Verfahren nach einem der vorhergehenden Ansprüche, wobei die Nachbarschaft eine Achter- Nachbarschaft ist.

10. Verfahren nach einem der vorhergehenden Ansprüche, wobei die Quelle dafür konfiguriert ist, die Fracht zur Materialunterscheidung mit mindestens zwei unterschiedlichen Energieniveaus zu bestrahlen, wobei das Verfahren zum Verarbeiten der Inspektionsdaten für jedes der mindestens zwei verschiedenen Energieniveaus durchgeführt wird.

11. Verfahren nach einem der vorhergehenden Ansprüche, wobei der absolute Wert des Gradienten entlang der Richtung x derart ist, dass:

$$\left|\nabla_{x,i,j}\right| = \left|I_{i+1,j} - I_{i-1,j}\right|/2$$

wobei $I_{i,j}$ eine Intensität eines Pixels (i,j) ist, wobei i die Koordinate in der Richtung x ist, die der wechselseitigen Scan-Verschiebung entspricht, und/oder
der absolute Wert des Gradienten basierend auf einer Sobel-Maske berechnet wird.

12. Verfahren nach einem der vorhergehenden Ansprüche, wobei der festgelegte Schwellenwert ein konstanter Wert ist oder von einem Pixel zu einem anderen Pixel variiert.

13. System, Folgendes umfassend:

einen Prozessor; und
einen Speicher, der Anweisungen speichert, die bei Ausführung durch den Prozessor den Prozessor in die Lage versetzen, ein Verfahren zur Verarbeitung von Inspektionsdaten durchzuführen, die mit einer Fracht in Verbindung stehen, die durch eine Vielzahl M aufeinanderfolgender Impulse von Röntgenstrahlen bestrahlt wird, Folgendes umfassend:
Erhalten der Inspektionsdaten,

wobei die Inspektionsdaten im Ergebnis des Scannens der Fracht mit Hilfe einer Matrix generiert werden, welche eine Vielzahl N von mindestens zwei Reihen aus Detektoren und eine Quelle der Vielzahl M aufeinanderfolgender Impulse umfasst, wobei sich die Matrix in einem Abstand D zu der Quelle befindet, wobei das Scannen Folgendes umfasst:

wechselseitiges Verschieben der Fracht und der Matrix mit einer wechselseitigen Scan-Verschiebung, und

Detektieren, mit der Matrix, von Strahlung, die der Vielzahl M aufeinanderfolgender Röntgenstrahl-Impulse, welche die Fracht bestrahlen, entspricht, während der wechselseitigen Scan-Verschiebung,

wobei in den Inspektionsdaten Strahlung, die der Vielzahl M aufeinanderfolgender Impulse, welche die Fracht bestrahlen und durch die Vielzahl N von mindestens zwei Reihen aus Detektoren detektiert werden, in einer ersten Reihenfolge angeordnet sind, die einer Ebene der Matrix in einer Richtung entspricht, die der wechselseitigen Scan-Verschiebung entspricht;

Bestimmen einer oder mehrerer aufeinanderfolgender Rekonstruktionszonen für die Inspektionsdaten, wobei jede Rekonstruktionszone einem Bereich von Abständen zu der Quelle entspricht, in dem Strahlung, die der Vielzahl M aufeinanderfolgender Impulse, welche die Fracht bestrahlen, in einer Reihenfolge angeordnet ist, die sich von der ersten Reihenfolge unterscheidet und sich von der einer anderen Rekonstruktionszone der einen oder mehreren aufeinanderfolgenden Rekonstruktionszonen unterscheidet;

Auswählen einer oder mehrerer Rekonstruktionsebenen, basierend auf der bestimmten einen oder den bestimmten mehreren Rekonstruktionszonen;

für jede ausgewählte Rekonstruktionsebene Generieren eines Zwischenbildes der Fracht;

Generieren eines Durchschnittsbildes durch Mitteln aller generierten Zwischenbilder;

auf dem generierten Durchschnittsbild Auswählen eines oder mehrerer Pixel, die in einer Richtung, die der wechselseitigen Scan-Verschiebung entspricht, einen Gradienten aufweisen, dessen absoluter Wert größer als ein festgelegter Schwellenwert ist; und

für jedes der ausgewählten Pixel:

Extrahieren einer Nachbarschaft des ausgewählten Pixels von jedem generierten Zwischenbild, und Bestimmen, in den extrahierten Nachbarschaften, der Nachbarschaft, die im Vergleich mit den anderen extrahierten Nachbarschaften ein Kriterium minimiert.

14. System nach dem vorhergehenden Anspruch, wobei der Speicher Anweisungen speichert, die bei Ausführung durch den Prozessor den Prozessor in die Lage versetzen, das Verfahren nach einem der Ansprüche 2 bis 12 durchzuführen.

15. Computerprogrammprodukt oder Computerprogramm, das Anweisungen umfasst., die bei Ausführung durch den Prozessor den Prozessor in die Lage versetzen, das Verfahren nach einem der Ansprüche 1 bis 12 durchzuführen oder das System nach Anspruch 13 oder 14 bereitzustellen.

**Revendications**

1. Procédé mis en œuvre par ordinateur permettant de traiter des données d'inspection associées à une cargaison irradiée par une pluralité M d'impulsions successives de rayons X, comprenant :

l'obtention des données d'inspection,

les données d'inspection étant générées à la suite du balayage de la cargaison à l'aide d'une matrice comprenant une pluralité N d'au moins deux rangées de détecteurs, et une source de la pluralité M d'impulsions successives, la matrice étant située à une distance D de la source, le balayage comprenant :

le déplacement mutuel de la cargaison et de la matrice avec un déplacement de balayage mutuel, et la détection, à l'aide de la matrice, du rayonnement correspondant à la pluralité M d'impulsions successives de rayons X irradiant la cargaison, au cours du déplacement de balayage mutuel,

dans lequel, dans les données d'inspection, le rayonnement correspondant à la pluralité M d'impulsions successives irradiant la cargaison et détecté par la pluralité N d'au moins deux rangées de détecteurs est disposé dans un premier ordre correspondant à un niveau de la matrice, dans une direction correspondant au déplacement de balayage mutuel ;

la détermination d'une ou plusieurs zones de reconstruction successives pour les données d'inspection, dans lequel chaque zone de reconstruction correspond à une plage de distances par rapport à la source dans laquelle le rayonnement correspondant à la pluralité M d'impulsions successives irradiant la cargaison est disposé dans un ordre différent du premier ordre et différent de celui d'une autre zone de reconstruction parmi la ou les zones de reconstruction successives ;

la sélection d'un ou plusieurs plans de reconstruction, sur la base de la ou des zones de reconstruction déterminées ;

pour chaque plan de reconstruction sélectionné, la génération d'une image intermédiaire de la cargaison ;

la génération d'une image moyenne en faisant la moyenne de toutes les images intermédiaires générées ;

sur l'image moyenne générée, la sélection d'un ou plusieurs pixels ayant un gradient, dans une direction correspondant au déplacement de balayage mutuel, supérieur en valeur absolue à un seuil prédéterminé ; et

pour chacun des pixels sélectionnés :

l'extraction d'un voisinage du pixel sélectionné à partir de chaque image intermédiaire générée, et

la détermination, dans les voisinages extraits, du voisinage qui minimise un critère par rapport aux autres voisinages extraits.

2. Procédé selon la revendication précédente, comprenant en outre la génération d'une image finale de la cargaison correspondant à une image moyenne corrigée, l'image finale comprenant des pixels et la génération de l'image finale comprenant :

l'affectation, pour chaque pixel de l'image finale correspondant à l'un des pixels sélectionnés, d'une valeur du pixel dans l'image intermédiaire générée correspondant au voisinage déterminé ; et

l'affectation, pour chaque autre pixel de l'image finale, d'une valeur du pixel dans l'image moyenne ou dans l'image intermédiaire générée correspondant à une zone de reconstruction la plus proche de la matrice.

3. Procédé selon l'une quelconque des revendications précédentes, comprenant en outre le redimensionnement de l'image finale générée, dans la direction correspondant au déplacement de balayage mutuel et/ou perpendiculairement à la direction correspondant au déplacement de balayage mutuel, pour obtenir un rapport largeur sur hauteur correspondant à un plan médian de la cargaison, le plan médian étant sensiblement parallèle à la matrice.

4. Procédé selon l'une quelconque des revendications précédentes, comprenant en outre, pour chaque pixel correspondant à l'un des pixels sélectionnés :

la détermination d'informations relatives à la zone de reconstruction déterminée correspondant au voisinage déterminé, optionnellement dans lequel la détermination des informations comprend en outre :

la prédétermination d'un nombre de zones de profondeur possibles, le nombre de zones de profondeur possibles étant égal ou inférieur au nombre de la ou des zones de reconstruction successives déterminées ;

la fusion de la ou des zones de reconstruction successives déterminées en une ou plusieurs zones de profondeur, un nombre de la ou des zones de profondeur correspondant au nombre de zones de profondeur possibles ;

l'affectation d'une zone de profondeur fusionnée à chaque pixel correspondant à l'un des pixels sélectionnés, sur la base des informations déterminées ; et

la génération d'une image de profondeur sur la base de l'affectation, chaque pixel ne correspondant pas à l'un des pixels sélectionnés n'étant pas affecté à une zone de profondeur fusionnée.

5. Procédé selon la revendication précédente, comprenant en outre la génération d'une image finale de la cargaison correspondant à une image moyenne corrigée, l'image finale comprenant des pixels et la génération de l'image finale comprenant :

l'affectation, pour chaque pixel de l'image finale correspondant à l'un des pixels sélectionnés, d'une valeur du pixel dans l'image intermédiaire générée correspondant au voisinage déterminé ; et

l'affectation, pour chaque autre pixel de l'image finale, d'une valeur du pixel dans l'image moyenne ou dans l'image intermédiaire générée correspondant à une zone de reconstruction la plus proche de la matrice, et comprenant en outre :

la superposition de l'image de profondeur générée sur l'image finale générée de la cargaison, optionnellement comprenant en outre :

le débruitage de l'image de profondeur générée en forçant des pixels adjacents à appartenir à une zone de profondeur commune,

optionnellement, le pixel correspondant à l'un des pixels sélectionnés comprend :

au moins un pixel de l'image moyenne générée, et/ou

au moins un pixel de l'image finale de la cargaison,

optionnellement, dans lequel les informations déterminées relatives à la zone de reconstruction déterminée comprennent :

un numéro ordinal correspondant à une position relative de la zone de reconstruction déterminée dans la ou les zones de reconstruction successives et/ou une zone de profondeur, chaque pixel ne correspondant pas à l'un des pixels sélectionnés n'étant pas associé à un numéro ordinal et/ou à une zone de profondeur ; et/ou

au moins une distance de la source correspondant à au moins une limite de la plage de distances associée à la zone de reconstruction déterminée dans la ou les zones de reconstruction successives.

6.  Procédé selon l'une quelconque des revendications précédentes, dans lequel un déplacement de balayage mutuel $\delta$ entre deux impulsions est une constante pendant le déplacement de balayage mutuel, et dans lequel la détermination de la ou des zones de reconstruction successives pour les données d'inspection est basée sur une séquence ordonnée de la position de rayonnement X(k,i,z), le long d'une direction du déplacement de balayage mutuel, associée à une $i^{ème}$ rangée de détecteurs et à une distance z de la source, pour l'indice de pulsation k, de telle sorte que :

$$X(k,i,z)=(k+i.m(z)).\delta+i.r(z)$$

avec m étant un nombre entier,

r(z) appartenant à l'intervalle [0,$\delta$[, et

de sorte que m(z).$\delta$+r(z)=p.(z/D), p étant un pas entre la pluralité N d'au moins deux rangées de détecteurs.

7.  Procédé selon l'une quelconque des revendications 1 à 6, dans lequel un déplacement de balayage mutuel $\delta$ entre deux impulsions n'est pas une constante pendant le déplacement de balayage mutuel, et dans lequel la détermination de la ou des zones de reconstruction successives pour les données d'inspection est basée sur une séquence ordonnée de la position de rayonnement X(k,i,z), le long d'une direction du déplacement de balayage mutuel, correspondant à une $i^{ème}$ rangée de détecteurs et à une distance z de la source, pour l'indice de pulsation d'impulsion k, de telle sorte que :

$$X(k, i, z) = k. \delta(k) + i. p. \frac{z}{D} + \frac{p}{2}. (1 - \frac{z}{D})$$

avec p étant un pas entre la pluralité N d'au moins deux rangées de détecteurs, optionnellement comprenant en outre :

la détermination de X(k,i,z) avec k variant de 1 à M, itérativement à partir de z égal à D jusqu'à une distance prédéterminée hors d'une zone de balayage, chaque itération étant effectuée en utilisant un décrément prédéterminé itératif d ; et

pour chaque itération :

l'ordination de la X(k,i,z) déterminée,

la détermination du fait de savoir si l'ordination est différente d'une ordination dans une itération précédente, et

si l'ordination est différente de celle d'une ordination dans une itération précédente, la détermination d'une distance correspondante de la matrice comme limite d'une zone de reconstruction.

8.  Procédé selon l'une quelconque des revendications précédentes, dans lequel la détermination du voisinage ayant le critère minimal comprend :

pour chaque valeur $I_{ij,p}$ de la $p^{ième}$ image intermédiaire générée Ip, dans des coordonnées (i,j) correspondant à l'un des pixels sélectionnés, et pour l'ensemble de l'image intermédiaire générée Ip, la détermination de l'énergie E de telle sorte que :

$$E\left(I_{ij,p}\right) = (\nabla_x I)^2{}_{ij,p} + g(I_{ij,p})$$

avec un premier terme étant un gradient le long de la direction x du déplacement

de balayage mutuel, au carré, et

un second terme étant une fonction de pénalisation g configurée pour pénaliser, dans la minimisation, des voisinages ayant des niveaux de gris différents ; et

la sélection du voisinage associé à l'image intermédiaire générée Ip ayant une énergie déterminée minimale, optionnellement dans lequel g est une fonction de Geman-McClure définie par :

$$g\left(I_{ij,p}\right) = \sum_{k \in N_{ij}^6} \rho(r_k, \sigma(r_k))$$

avec $N_{ij}^6$ étant le voisinage du pixel situé en (i,j), indexé par k de 1 à 6, et dont des pixels constitutifs sont les trois pixels les plus proches de (i,j) situés dans la colonne précédente et les trois pixels les plus proches de (i,j) situés dans la colonne suivante,

$$\rho(r, \sigma) = \frac{r^2}{r^2 + \sigma^2},$$

$r_k = \left\{ I_k - I_{ij} \, \middle| \, k \in N_{ij}^6 \right\}$, $I_{ij}$ étant la valeur d'image au niveau du pixel (i,j) et $I_k$ les valeurs d'image pour le pixel dans le voisinage $N_{ij}^6$, et

$\sigma(r_k)$ est l'écart-type de la valeur de $r_k$ sur le voisinage.

9. Procédé l'une quelconque des revendications précédentes, dans lequel le voisinage est un voisinage à 8 connexions.

10. Procédé l'une quelconque des revendications précédentes, dans lequel la source est configurée pour irradier la cargaison avec au moins deux niveaux d'énergie différents pour la discrimination des matériaux, le procédé de traitement des données d'inspection étant effectué pour chacun des au moins deux niveaux d'énergie différents.

11. Procédé l'une quelconque des revendications précédentes, dans lequel la valeur absolue du gradient le long de la direction x est telle que :

$$\left| \nabla_{x,i,j} \right| = \left| I_{i+1,j} - I_{i-1,j} \right| / 2$$

avec $I_{i,j}$ étant l'intensité d'un pixel (i,j), avec i étant la coordonnée dans la direction x correspondant au déplacement de balayage mutuel, et/ou
la valeur absolue du gradient est calculée sur la base d'un masque de Sobel.

12. Procédé l'une quelconque des revendications précédentes, dans lequel le seuil prédéterminé est une valeur constante ou varie d'un pixel à l'autre.

13. Système comprenant :

un processeur ; et
une mémoire stockant des instructions qui, lorsqu'elles sont exécutées par le processeur, amènent le processeur à mettre en œuvre un procédé de traitement de données d'inspection associées à une cargaison irradiée par une pluralité M d'impulsions successives de rayons X, comprenant :
l'obtention des données d'inspection,

les données d'inspection étant générées à la suite du balayage de la cargaison à l'aide d'une matrice comprenant une pluralité N d'au moins deux rangées de détecteurs, et une source de la pluralité M d'impulsions successives, la matrice étant située à une distance D de la source, le balayage comprenant :

le déplacement mutuel de la cargaison et de la matrice avec un déplacement de balayage mutuel, et

la détection, à l'aide de la matrice, du rayonnement correspondant à la pluralité M d'impulsions successives de rayons X irradiant la cargaison,

au cours du déplacement par balayage mutuel,

dans lequel, dans les données d'inspection, le rayonnement correspondant à la pluralité M d'impulsions successives irradiant la cargaison et détecté par la pluralité N d'au moins deux rangées de détecteurs est disposé dans un premier ordre correspondant à un niveau de la matrice, dans une direction correspondant au déplacement de balayage mutuel ;

la détermination d'une ou plusieurs zones de reconstruction successives pour les données d'inspection, dans lequel chaque zone de reconstruction correspond à une plage de distances par rapport à la source dans laquelle le rayonnement correspondant à la pluralité M d'impulsions successives irradiant la cargaison est disposé dans un ordre différent du premier ordre et différent de celui d'une autre zone de reconstruction parmi la ou les zones de reconstruction successives ;

la sélection d'un ou plusieurs plans de reconstruction, sur la base de la ou des zones de reconstruction déterminées ;

pour chaque plan de reconstruction sélectionné, la génération d'une image intermédiaire de la cargaison ;

la génération d'une image moyenne en faisant la moyenne de toutes les images intermédiaires générées ;

sur l'image moyenne générée, la sélection d'un ou plusieurs pixels ayant un gradient, dans une direction correspondant au déplacement de balayage mutuel, supérieur en valeur absolue à un seuil prédéterminé ; et

pour chacun des pixels sélectionnés :

l'extraction d'un voisinage du pixel sélectionné à partir de chaque image intermédiaire générée, et

la détermination, dans les voisinages extraits, du voisinage qui minimise un critère par rapport aux autres voisinages extraits.

14. Système selon la revendication précédente, dans lequel la mémoire stocke des instructions qui, lorsqu'elles sont exécutées par le processeur, amènent le processeur à mettre en œuvre le procédé selon l'une quelconque des revendications 2 à 12.

15. Produit programme d'ordinateur ou programme d'ordinateur comprenant des instructions qui, lorsqu'elles sont exécutées par un processeur, amènent le processeur à mettre en œuvre le procédé selon l'une quelconque des revendications 1 à 12 ou de fournir le système selon la revendication 13 ou 14.

Figure 1B

Figure 1A

Figure 2

Figure 3

Figure 4

Figure 5B

Figure 5A

$N_{ij}^6$

Pixel Iij,p

Figure 6

S7

Generating final image — S8

Resizing final image — S9

Determining information

S10

Superimposing depth image on final image — S11

Figure 7

EP 4 073 744 B1

Figure 8

Figure 9

**REFERENCES CITED IN THE DESCRIPTION**

**Patent documents cited in the description**

- US 2004251415 A1